# EUROPÄISCHE PATENTANMELDUNG

(11) **EP 4 056 905 A1**
(43) Veröffentlichungstag der Anmeldung: **14.09.2022**
(21) Anmeldenummer: 22159637.2
(22) Anmeldetag: 02.03.2022
(51) Int. Cl.: F24C 15/20, A61L 9/20

(54) **DUNSTABZUGSVORRICHTUNG**

(30) Priorität: 08.03.2021 DE 102021105508; 01.12.2021 DE 202021106566 U
(71) Anmelder: Berbel Ablufttechnik GmbH, 48432 Rheine (DE)
(72) Erfinder: Robusch, Stephan, 48291 Telgte (DE); Üffing, Stefan, 48496 Hopsten (DE)
(74) Vertreter: Schneiders & Behrendt Bochum

(57) **Zusammenfassung**

Die Erfindung betrifft eine Dunstabzugsvorrichtung (1) zum Abzug von Kochdünsten mittels eines Luftstromes (2), mit einem Gehäuse (3), das wenigstens eine Luftansaugöffnung (4) und wenigstens einen Luftauslass (5) für den Luftstrom (2) aufweist, wenigstens einem in dem Gehäuse (3) angeordneten Lüfter (6) zur Erzeugung des Luftstromes (2), und wenigstens einem in dem Gehäuse (3) in dem Luftstrom (2) zwischen Luftansaugöffnung (4) und Lüfter (6) angeordneten Abscheideelement (7) zur Trennung eines oder mehrerer Bestandteile, insbesondere Fett und Öl, von den Kochdünsten, wobei in oder an dem Gehäuse (3) mindestens eine UVC-Lichtquelle (8) angeordnet ist, die eingerichtet ist, den aus dem Lüfter (6) austretenden Luftstrom (2) zum Zwecke der Entkeimung des aus dem Lüfter (6) austretenden Luftstroms (2) und/oder Zwecke der Beseitigung organischer und geruchstragender Substanzen zu bestrahlen.

## Beschreibung

Die Erfindung betrifft eine Dunstabzugsvorrichtung zum Abzug von Kochdünsten mittels eines Luftstromes, mit einem Gehäuse, das wenigstens eine Luftansaugöffnung und wenigstens einen Luftauslass aufweist, wenigstens einem in dem Gehäuse angeordneten Lüfter zur Erzeugung des Luftstromes, und wenigstens einem in dem Gehäuse in dem Luftstrom zwischen Luftansaugöffnung und Lüfter angeordneten Abscheideelement zur Trennung eines oder mehrerer Bestandteile, insbesondere Fett und Öl, von den Kochdünsten.

Eine solche Dunstabzugsvorrichtung ist allgemein bekannt. Nachteilig an der bekannten Dunstabzugsvorrichtung ist, dass insbesondere Viren, vor allem Corona-Viren, wie Covid-19, und/oder organische und geruchstragende Substanzen ganz oder teilweise nicht von dem Abscheideelement und auch nicht von einem im Luftstrom nachgeschalteten Filter (z.B. Aktivkohlefilter) abgeschieden werden und sich insbesondere beim Umluftbetrieb der Dunstabzugsvorrichtung weiter im Raum bzw. in der Umgebung verteilen.

Es ist daher Aufgabe der Erfindung, eine verbesserte Dunstabzugsvorrichtung anzugeben, die eine einfache, effektive und fehlerunanfällige Entkeimung des aus dem Lüfter austretenden Luftstroms bzw. eine Beseitigung organischer und geruchstragender Substanzen ermöglicht.

Gelöst wird diese Aufgabe durch eine Dunstabzugsvorrichtung mit den Merkmalen des Anspruchs 1.

Dadurch, dass in oder an dem Gehäuse mindestens eine UVC-Lichtquelle angeordnet ist, die eingerichtet ist, den aus dem Lüfter austretenden Luftstrom zum Zwecke der Entkeimung des aus dem Lüfter austretenden Luftstroms und/oder Zwecke der Beseitigung organischer und geruchstragender Substanzen zu bestrahlen, kann der aus dem Lüfter austretende Luftstrom einfach und sicher gereinigt und sterilisiert werden. Die Bestrahlung des Luftstromes im Bereich hinter dem Lüfter bietet die Möglichkeit den vollständigen Luftstrom effektiv zu behandeln. So können insbesondere Viren, vor allem Corona-Viren, wie Covid-19, oder organische und geruchstragende Substanzen, die nicht über das Abscheideelement abgeschieden wurden inaktiviert oder reduziert werden. Die Bestrahlung des aus dem Lüfter austretenden Luftstromes führt zu einer sicheren Entkeimung des Luftstromes nach Austritt aus dem Lüfter und somit vor der Abgabe des Luftstromes an die Umgebung über den Luftauslass im Gehäuse. Mit der Bestrahlung des aus dem Lüfter austretende Luftstroms wird eine chemische Reaktion der mit den Kochdünsten eingesauten organischen und geruchstragenden Substanzen erreicht, die zu einer Zersetzung von Geruchsstoffen und damit zu einer Geruchsreduktion führt. Hierdurch lässt sich auch das Adsorptionsvermögen im Luftstrom nachgeschalteter Geruchsfilter verbessern.

Vorteilhafte Ausgestaltungen und Weiterbildungen der Erfindung ergeben sich aus den abhängigen Ansprüchen. Es ist darauf hinzuweisen, dass die in den Ansprüchen einzeln aufgeführten Merkmale auch in beliebiger und technologisch sinnvoller Weise miteinander kombiniert werden können und somit weitere Ausgestaltungen der Erfindung aufzeigen.

Gemäß einer vorteilhaften Ausgestaltung der Erfindung ist vorgesehen, dass der Luftstrom in dem von der mindestens einen UVC-Lichtquelle bestrahlten Bereich zur Verlangsamung des Luftstroms mit einem vergrößerten Querschnitt geführt ist. Durch den vergrößerten Querschnitt lässt sich der Luftstrom einfach verlangsamen, sodass eine effektive, d.h. über einen hinreichenden Zeitraum andauernde Behandlung des Luftstromes durch die mindestens eine UVC-Lichtquelle in dem bestrahlten Bereich möglich ist.

Besonders bevorzugt ist eine Ausführungsform, die vorsieht, dass mehrere UVC-Lichtquellen dazu eingerichtet sind, den Luftstrom zu bestrahlen. Mit mehreren UVC-Lichtquellen lässt sich der Luftstrom besonders gut behandeln, sodass insbesondere Viren, vor allem Corona-Viren, wie Covid-19, oder andere Keime zuverlässig abgetötet werden. Die mehreren UVC-Lichtquellen können dazu eingerichtet sein, einen Abschnitt des Luftstroms zwischen Lüfter und Luftauslass zu bestrahlen. Über die Anordnung von mehreren UVC-Lichtquellen, die einen Abschnitt des Luftstroms zwischen Lüfter und Luftauslass bestrahlen, kann eine effektive und vollständige Behandlung des Luftstromes durch die UVC-Lichtquellen sichergestellt werden. Die mehreren UVC-Lichtquellen können auch dazu eingerichtet sein, einen Abschnitt des Luftstroms zwischen dem Luftauslass und einem Filterelement für den Umluftbetrieb zu bestrahlen. Über die Anordnung von mehreren UVC-Lichtquellen, die einen Abschnitt des Luftstroms zwischen dem Luftauslass und dem Filterelement bestrahlen, kann eine effektive und vollständige Behandlung des Luftstromes durch die UVC-Lichtquellen sichergestellt werden. Hierdurch lassen sich insbesondere Viren, vor allem Corona-Viren, wie Covid-19, oder organische und geruchstragende Substanzen im aus dem Lüfter austretenden Luftstrom effektiv und vollständig inaktivieren oder zersetzen.

Eine besonders vorteilhafte Ausführung der Erfindung bezieht sich darauf, dass die UVC-Lichtquellen in einer umfangsseitig geschlossenen, von dem Luftstrom durchströmten Entkeimungszelle angeordnet sind. Die umfangsseitig geschlossene Entkeimungszelle ermöglicht eine zuverlässige Entkeimung und Zersetzung organischer und geruchstragender Substanzen in dem aus dem Lüfter austretenden Luftstrom. Die Durchströmung des Luftstromes durch eine umfangsseitig geschlossene Entkeimungszelle, die mit UVC-Lichtquellen, bevorzugt umfangsseitig, bestückt ist, sorgt für eine zuverlässige Entkeimung und Zersetzung organischer und geruchstragender Substanzen in dem aus dem Lüfter austretenden Luftstrom.

Eine besonders vorteilhafte Ausführung der Erfindung sieht vor, dass die UVC-Lichtquellen auf einem von dem Luftstrom durchströmten, die Entkeimungszelle innerhalb eines begrenzenden Kreisring oder Zylinder angeordnet sind. Mit der Anordnung der UCV-Lichtquellen auf einem die Entkeimungszelle innerhalb des begrenzenden Kreisring oder Zylinder kann eine vollständige Entkeimung und Zersetzung organischer und geruchstragender Substanzen in dem aus dem Lüfter austretenden Luftstrom erreicht werden. Eine gleichmäßige Bestrahlung des gesamten Luftstromes ist gewährleistet. Die UVC-Lichtquellen können auch auf einem von dem Luftstrom durchströmten, die Entkeimungszelle umfangsseitig begrenzenden Kreisring oder Zylinder angeordnet sein. Mit der Anordnung der UCV-Lichtquellen auf einem die Entkeimungszelle umfangsseitig begrenzenden Kreisring oder Zylinder kann eine vollständige Entkeimung und Zersetzung organischer und geruchstragender Substanzen in dem aus dem Lüfter austretenden Luftstrom erreicht werden. Eine gleichmäßige Bestrahlung des gesamten Luftstromes ist gewährleistet.

Eine vorteilhafte Ausführung der Erfindung sieht vor, dass mindestens ein die von der UVC-Lichtquelle emittierte UVC-Strahlung reflektierender Reflektor in dem Gehäuse angeordnet ist. Über die Reflektion der von der UVC-Lichtquelle emittierten UVC-Strahlung an dem mindestens einen Reflektor kann die Bestrahlung des aus dem Lüfter austretenden Luftstromes in dem Gehäuse besonders effektiv erfolgen. Insbesondere wird vermieden, dass ein großer Anteil des UVC-Lichts ohne Wirkung auf den Lichtstrom absorbiert wird. Besonders vorteilhaft ist dabei eine Ausführungsform, die vorsieht, dass der Reflektor gegenüber mindestens einer UVC-Lichtquelle angeordnet ist, wobei der Luftstrom zur Entkeimung zwischen dem Reflektor und der gegenüberliegenden UVC-Lichtquelle geführt ist.

Eine vorteilhafte Ausgestaltung sieht vor, dass der Lüfter eine Ansaugöffnung und eine Ausblasöffnung aufweist, wobei mindestens eine UVC-Lichtquelle unmittelbar an der Ausblasöffnung angeordnet ist. Durch die Anordnung der UVC-Lichtquelle unmittelbar an der Ausblasöffnung des Lüfters kann auf einfache Weise eine vollständige und effektive Behandlung des austretenden Luftstromes gewährleistet werden. Es wird sichergestellt, dass der gesamte Luftstrom zuverlässig von der UVC-Strahlung erreicht wird.

Eine besonders vorteilhafte Ausführung der Erfindung sieht vor, dass die mindestens eine UVC-Lichtquelle auf einer Platine verlötet in die Ausblasöffnung eingesetzt ist, wobei die UVC-Lichtquelle in Richtung des aus der Ausblasöffnung austretenden Luftstroms abstrahlt. Mit der Anordnung der UVC-Lichtquelle auf einer Platine ist eine besonders einfache Montage der UVC-Lichtquelle möglich, da die Platine einfach in die Ausblasöffnung eingesetzt werden kann. Hierdurch besteht die Möglichkeit, auch alte Dunstabzugsvorrichtungen mit UVC-Lichtquellen nachträglich auszurüsten. Die Platine umfasst bevorzugt alle zur Steuerung der UVC-Lichtquelle relevanten Elektronikkomponenten. Außerdem weist die Platine bevorzugt einen Steckverbinder zum Anschluss einer Spannungsversorgung der Dunstabzugsvorrichtung auf. Mit der Ausrichtung der Abstrahlrichtung in Richtung der Strömungsrichtung des aus der Ausblasöffnung austretenden Luftstromes kann der Luftstrom zuverlässig beim Ausströmen aus der Ausblasöffnung bestrahlt und Viren, vor allem Corona-Viren, wie Covid-19, oder organische und geruchstragende Substanzen im Luftstrom effektiv und vollständig inaktiviert oder zersetzt werden.

Besonders bevorzugt ist eine Ausführungsform, die vorsieht, dass die Platine an Rasthaken in der Ausblasöffnung verrastbar ist. Mit der Verrastung der Platine an Rasthaken in der Ausblasöffnung ist eine besonders einfache und schnelle, ggf. auch nachträgliche Montage der UVC-Lichtquelle in der Ausblasöffnung möglich.

Gemäß einer bevorzugten Ausgestaltung der Erfindung ist vorgesehen, dass dem Luftauslass ein den Luftstrom filterndes Filterelement, insbesondere ein Geruchsfilter, nachgeordnet ist. Über die Nutzung eines Filterelements am Luftauslass kann der aus dem Lüfter austretende Luftstrom nach der Bestrahlung effektiv von verbliebenen geruchstragenden Substanzen befreit werden. Durch die vorherige Bestrahlung des Luftstromes lässt sich auch das Adsorptionsvermögen der im Luftstrom nachgeschalteten Geruchsfilter verbessern. Denn ein alleiniger Einsatz beispielsweise eines mit Aktivkohle versehenen Geruchsfilters ist nur begrenzt geeignet zur Desodorierung des in die Raumluft zurückgeführten Luftstromes.

Eine vorteilhafte Ausführung der Erfindung sieht vor, dass die mindestens eine UVC-Lichtquelle dazu eingerichtet ist, das Filterelement von dem Luftauslass her zum Zwecke der Entkeimung des Filterelements und/oder zum Zwecke der Beseitigung organischer und geruchstragender Substanzen auf dem Filterelement zu bestrahlen. Die mindestens eine UVC-Lichtquelle bestrahlt das Filterelement am Luftauslass, um das Filterelement zu entkeimen und/oder um organische und geruchstragende Substanzen auf dem Filterelement zu beseitigen. Damit können zusätzlich Keime, Viren, Schimmelpilze etc. auf dem Filterelement vermieden werden.

Gemäß einer vorteilhaften Ausgestaltung der Erfindung ist vorgesehen, dass das Filterelement einen durch Filterwände des Filterelements gebildeten Innenraum aufweist, wobei die Filterwände dazu eingerichtet sind, den durch den Innenraum strömenden Luftstrom zu filtern, wobei die Filterwände des Innenraums und der Innenraum selbst von der mindestens einen UVC-Lichtquelle bestrahlt werden. Der Innenraum wird von dem Luftstrom durchströmt und die Filterwände filtern den Luftstrom hiernach. Sowohl die Filterwände des Innenraums als auch der Innenraum selbst werden von der mindestens einen UVC-Lichtquelle zum Zwecke der Entkeimung von Innenraum und Luftstrom bestrahlt. Auch organische und geruchstragende Substanzen können so durch Bestrahlung aus dem Innenraum und aus dem durch den Innenraum strömenden Luftstrom beseitigt werden. In dem Innenraum des Filterelements kann der Luftstrom durch einen vergrößerten Querschnitt einfach verlangsamt werden, sodass eine effektive, d.h. über einen hinreichenden Zeitraum andauernde Behandlung des Luftstromes durch die mindestens eine UVC-Lichtquelle in dem bestrahlten Innenraum möglich ist. Das dabei auf die Filterwände abgestrahlte Licht der UVC-Lichtquelle sorgt zusätzlich für eine Entkeimung der Filterwände und zu einer Beseitigung von aus dem Luftstrom heraus gefilterten organischen und geruchstragenden Substanzen auf den Filterwänden im Innenraum.

Besonders vorteilhaft ist eine Ausführungsform, die vorsieht, dass die mindestens eine UVC-Lichtquelle eine UVC-Strahlung emittierende Leuchtdiode ist. Mit einer Leuchtdiode als emittierende UVC-Lichtquelle kann die Bestrahlung des aus dem Lüfter austretenden Luftstroms mit hinreichender Leistung besonders effektiv und energiesparend erfolgen.

Eine besonders vorteilhafte Ausführung der Erfindung sieht vor, dass die mindestens eine UVC-Lichtquelle eine UVC-Strahlung mit einer Wellenlänge von 100 bis 280 nm emittiert. Mit einer Wellenlänge in dem angegeben Bereich können Bakterien und Viren, vor allem Corona-Viren, wie Covid-19, in dem aus dem Lüfter austretenden Luftstrom besonders effektiv inaktiviert werden.

Gemäß einer vorteilhaften Ausgestaltung der Erfindung ist vorgesehen, dass die mindestens eine UVC-Lichtquelle dazu ausgelegt ist, in dem aus dem Lüfter austretenden Luftstrom Ozon zu erzeugen. Über die Erzeugung von Ozon in dem aus dem Lüfter austretenden Luftstrom kann eine Zersetzung organischer und geruchstragender Substanzen besonders effektiv erreicht werden, die Entkeimung wird durch den Kontakt der in dem Luftstrom enthaltenen Bakterien und Viren mit dem erzeugten Ozon weiter verbessert.

Weitere Merkmale, Einzelheiten und Vorteile der Erfindung ergeben sich aufgrund der nachfolgenden Beschreibung sowie anhand der Zeichnungen, die Ausführungsbeispiele der Erfindung zeigen. Es zeigt:
- Figur 1: erfindungsgemäße Dunstabzugsvorrichtung,
- Figur 2: weitere erfindungsgemäße Dunstabzugsvorrichtung,
- Figur 3: Schnittansicht durch Dunstabzugsvorrichtung,
- Figur 4: Schnittansicht durch Filterelement,
- Figur 5: weitere Schnittansicht durch Filterelement,
- Figur 6: weitere Schnittansicht durch Filterelement,
- Figur 7: Ansicht auf Ausblasöffnung,
- Figur 8: Detailansicht auf Ausblasöffnung, und
- Figur 9: Detailansicht auf Platine.

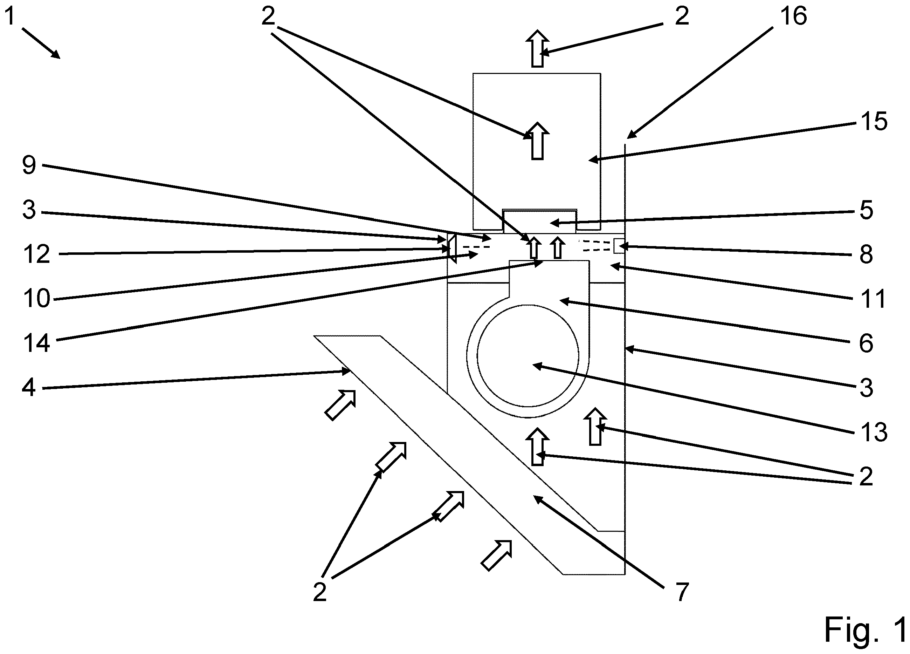

In der Figur 1 mit dem Bezugszeichen 1 bezeichnet ist eine erfindungsgemäße Dunstabzugsvorrichtung schematisch in einer Schnittansicht dargestellt. Die Dunstabzugsvorrichtung 1 ist in dem Ausführungsbeispiel als Dunstabzugshaube nach Art einer sogenannten Kopf-Frei-Haube ausgebildet. Die Dunstabzugsvorrichtung 1 kann aber mit gleichen Vorteilen auch als eine andere Art von Dunstabzugsvorrichtung 1, beispielsweise als Kochfeldabzug mit einem Abzug der Kochdünste nach unten ausgebildet sein. Die hier gezeigte Dunstabzugsvorrichtung 1 hingegen wird zum Abzug von Kochdünsten mittels eines Luftstromes 2 über einem Kochfeld angeordnet, auf dem die Kochdünste beim Kochen von Kochgut entstehen und nach oben aufsteigen. Die Dunstabzugshaube 1 verfügt über ein an der Wand 16 montierbares Gehäuse 3, das wenigstens eine Luftansaugöffnung 4 und wenigstens einen Luftauslass 5 für den Luftstrom 2, der in Figur 1 durch Blockpfeile dargestellt ist, aufweist. In dem Gehäuse 3 ist ein Lüfter 6 angeordnet, über den der Luftstrom 2 erzeugt wird. Der Lüfter 6 ist vorzugsweise ein elektrisch angetriebenes Gebläse, bevorzugt ein Radiallüfter.

In dem von dem Lüfter 6 erzeugten Luftstrom 2 ist zwischen der Luftansaugöffnung 4 und dem Lüfter 6 mindestens ein Abscheideelement 7 zur Trennung eines oder mehrerer Bestandteile, insbesondere Fett und Öl, von den Kochdünsten, angeordnet. Weiterhin ist in dem Gehäuse 3 mindestens eine UVC-Lichtquelle 8 angeordnet, die eingerichtet ist, den aus dem Lüfter 6 austretenden Luftstrom 2 zum Zwecke der Entkeimung des aus dem Lüfter 6 austretenden Luftstroms 2 und/oder Zwecke der Beseitigung organischer und geruchstragender Substanzen zu bestrahlen. Über die Bestrahlung des gesamten Luftstromes 2 in dem Bereich unmittelbar hinter dem Lüfter 6 ist eine besonders einfache Möglichkeit gegeben, den Luftstrom 2 effektiv und vollständig zu behandeln. So können insbesondere Viren, vor allem Corona-Viren, wie Covid-19, oder organische und geruchstragende Substanzen, die nicht über das Abscheideelement 7 abgeschieden wurden, inaktiviert oder reduziert werden. Der Luftstrom 2 wird hierzu vorteilhafterweise hinter dem Lüfter 6 in einen mit der UVC-Lichtquelle 8 bestrahlten Bereich geführt, der zur Verlangsamung des Luftstroms 2 einen vergrößerten Querschnitt 9 aufweist. So kann die Verweildauer der Luft des Luftstromes 2 im bestrahlten Bereich erhöht werden, sodass eine effektive Entkeimung mit vergleichsweise geringer Lichtleistung der UVC-Lichtquelle möglich ist. Vorteilhafterweise können auch mehrere UVC-Lichtquellen 8 dazu eingerichtet sind, einen Abschnitt 10 des Luftstroms 2 zwischen Lüfter 6 und Luftauslass 5 zu bestrahlen. Die UVC-Lichtquellen 8 sind bevorzugt in einer umfangsseitig geschlossenen, von dem Luftstrom 2 durchströmten Entkeimungszelle 11 angeordnet. Diese Entkeimungszelle 11 kann als rechteckiger Raum, wie im Ausführungsbeispiel gezeigt, ausgebildet sein, oder aber vorteilhafterweise auch als umfangsseitig begrenzter Kreisring oder Zylinder ausgebildet sein, auf dem die UVC-Lichtquellen 8 umfangsseitig angeordnet sind. Wie in Figur 1 zu erkennen, kann in dem Gehäuse 3, bevorzugt in der Entkeimungszelle 11, mindestens ein die von der UVC-Lichtquelle 8 emittierte UVC-Strahlung reflektierender Reflektor 12 angeordnet sein. Mit der Reflektion der emittierten UVC-Strahlung kann eine noch effektivere Behandlung des Luftstromes 2 erfolgen. Besonders effektiv ist die Bestrahlung des Luftstromes 2, wenn der Reflektor 12, wie in Figur 1 gezeigt, gegenüber der mindestens einen UVC-Lichtquelle 8 angeordnet ist und der Luftstrom 2 zur Entkeimung zwischen dem Reflektor 12 und der gegenüberliegenden UVC-Lichtquelle 8 durch das Gehäuse 3 geführt ist. Um eine besonders effektive Bestrahlung des Luftstromes 2 zu erreichen, ist die mindestens eine UVC-Lichtquelle 8 unmittelbar an der Ausblasöffnung 14 des Lüfters 6 angeordnet. So kann der gesamte Luftstrom 2, der über die Ansaugöffnung 13 des Lüfters 6 angesaugt wird, und über die Ausblassöffnung 14 ausgeblasen wird, vollständig von der UVC-Lichtquelle 8 bestahlt werden. Hierdurch lassen sich insbesondere Viren, vor allem Corona-Viren, wie Covid-19, oder organische und geruchstragende Substanzen im aus dem Lüfter 6 über die Ausblassöffnung 14 austretenden Luftstrom 2 effektiv und vollständig inaktivieren oder zersetzen. An dem Luftauslass 5 ist zudem ein den Luftstrom 2 filterndes Filterelement 15, insbesondere ein Geruchsfilter, nachgeordnet. Mit dem Filterelement 15 am Luftauslass 5 kann der aus dem Lüfter 6 austretende Luftstrom 2 nach der Bestrahlung durch die UVC-Lichtquelle 8 anschließend noch zusätzlich effektiv von letzten geruchstragende Substanzen befreit werden. Die vorherige Bestrahlung des Luftstromes 2 mit der UVC-Lichtquelle 8 ermöglicht das Adsorptionsvermögen des Geruchsfilters 15 zu verbessern. Der alleinige Einsatz beispielsweise eines mit Aktivkohle versehenen Geruchsfilters 15 ist nur begrenzt geeignet zur Desodorierung des in die Raumluft zurückgeführten Luftstromes 2, da die Fähigkeit der Aktivkohle geruchstragender Substanzen zu binden begrenzt ist.

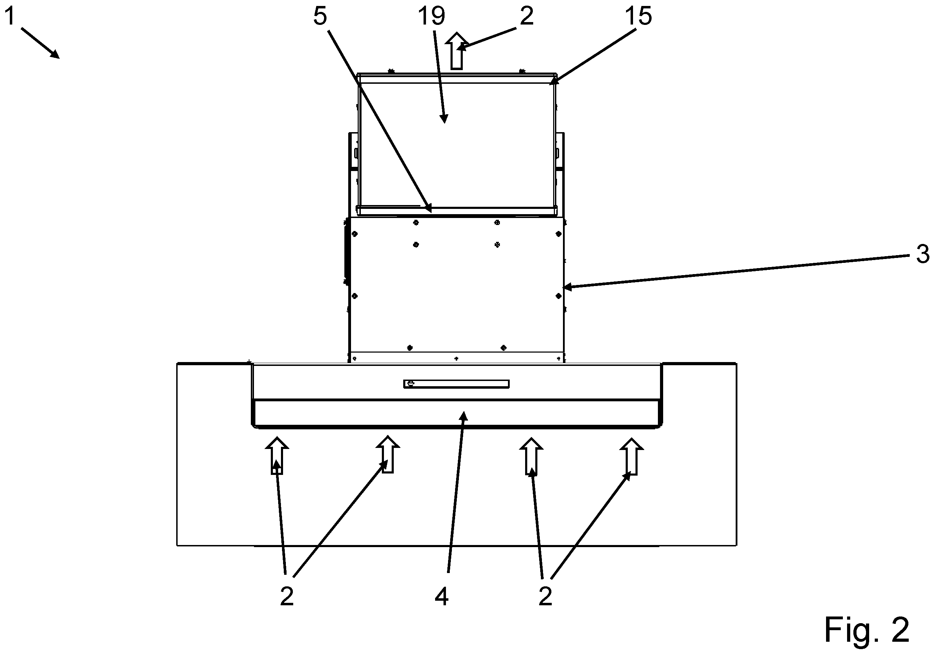

Die Figur 2 zeigt eine weitere erfindungsgemäße Dunstabzugsvorrichtung 1 in einer Frontansicht. Die Dunstabzugsvorrichtung 1 ist in dem Ausführungsbeispiel ebenfalls als Kopf-Frei-Haube ausgebildet. Die im Rahmen dieses Ausführungsbeispiels erläuterten Vorteile lassen sich aber auch bei einer anderen Art von Dunstabzugsvorrichtungen, beispielsweise bei einem Kochfeldabzug mit einem Abzug der Kochdünste nach unten, erreichen. Die in Figur 2 abgebildete Dunstabzugsvorrichtung 1 wird zum Abzug von Kochdünsten mittels eines Luftstromes 2 üblicherweise über einem Kochfeld angeordnet, auf dem die Kochdünste beim Kochen von Kochgut entstehen und durch die Thermik nach oben steigen. Die Dunstabzugsvorrichtung 1 weist ein Gehäuse 3 auf, das über wenigstens eine Luftansaugöffnung 4 und wenigstens einen Luftauslass 5 für den Luftstrom 2 verfügt. Der Luftstrom 2 ist in Figur 2 schematisch durch Blockpfeile angedeutet.

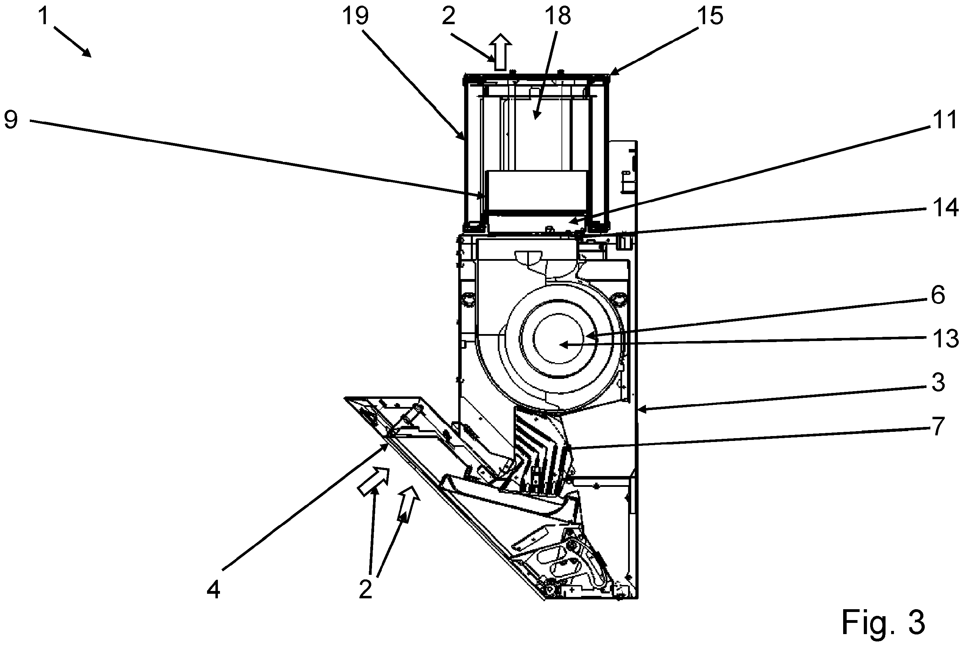

In Figur 3 ist eine seitliche Schnittansicht durch die Dunstabzugsvorrichtung 1 gemäß Figur 2 gezeigt. Hier ist zu erkennen, dass in dem Gehäuse 3 ein Lüfter 6 angeordnet ist, über den der Luftstrom 2 erzeugt wird. Der Lüfter 6 ist vorzugsweise ein elektrisch angetriebenes Gebläse, bevorzugt ein Radiallüfter. Zwischen der Luftansaugöffnung 4 und dem Lüfter 6 ist mindestens ein Abscheideelement 7 zur Trennung eines oder mehrerer Bestandteile, insbesondere Fett und Öl, von den Kochdünsten in dem von dem Lüfter 6 erzeugten Luftstrom 2 angeordnet. An dem Luftauslass 5 ist außerdem ein den Luftstrom 2 filterndes Filterelement 15, insbesondere ein Geruchsfilter nachgeordnet. Über das Filterelement 15 am Luftauslass 5 werden aus dem Luftstrom 2 letzte geruchstragende Substanzen beseitigt.

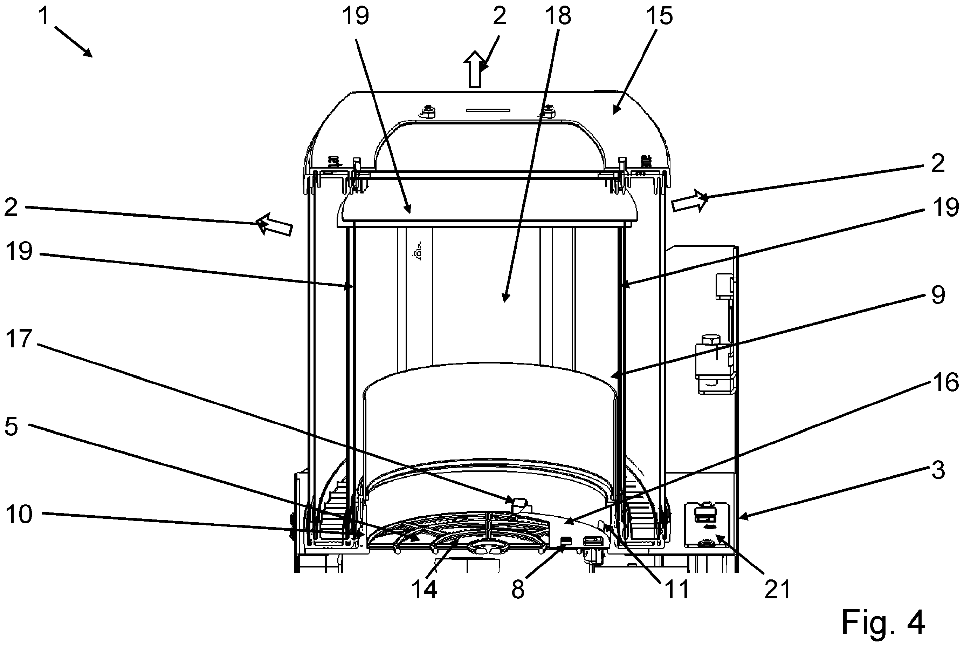

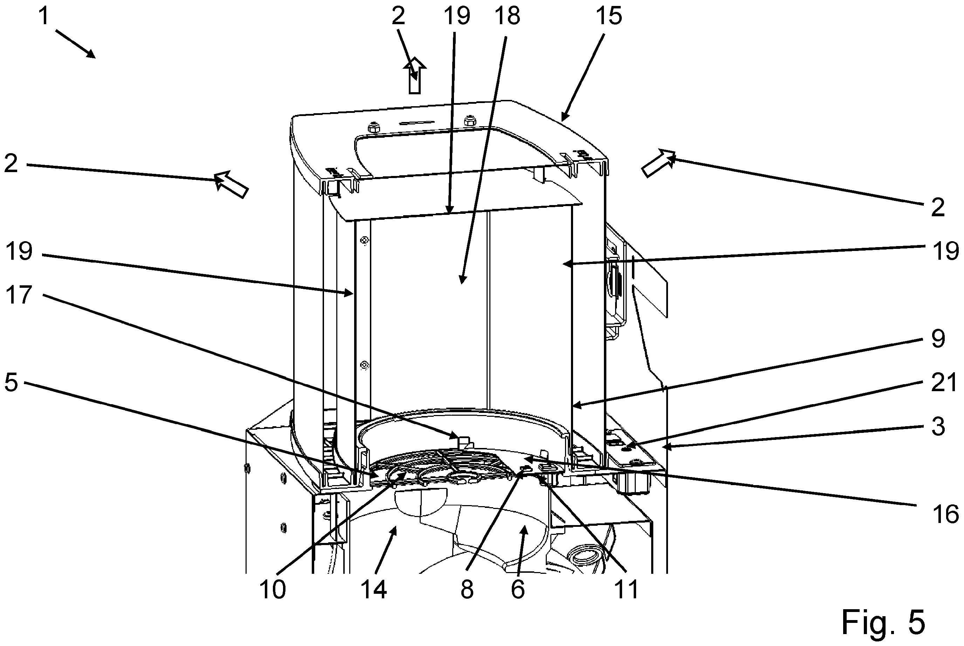

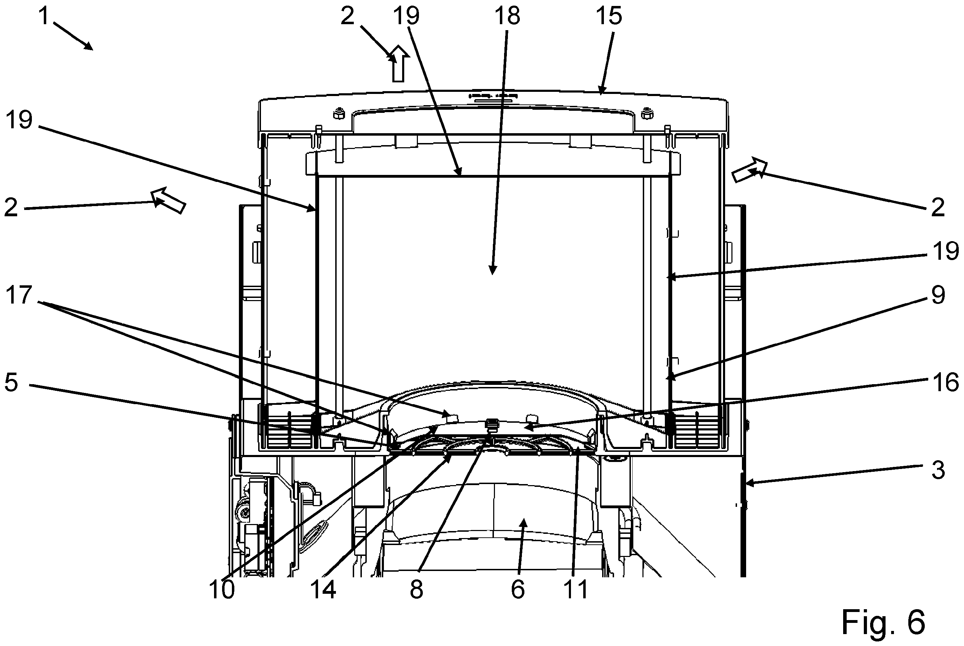

Die Figuren 4, 5 und 6 zeigen Schnittansichten durch das Filterelement 15 gemäß den Figuren 2 und 3. Neben dem Filterelement 15 ist in diesen Darstellungen jeweils ebenfalls der Luftauslass 5 des Gehäuses 3 zu sehen, der in diesem bevorzugten Ausführungsbeispiel unmittelbar an die Ausblasöffnung 14 des Lüfters 6 anschließt. In diesen Ansichten ist auch zu erkennen, dass an dem Gehäuse 3 mindestens eine UVC-Lichtquelle 8 angeordnet ist, die eingerichtet ist, den aus dem Lüfter 6 austretenden Luftstrom 2 zum Zwecke der Entkeimung des aus dem Lüfter 6 austretenden Luftstroms 2 und/oder Zwecke der Beseitigung organischer und geruchstragender Substanzen zu bestrahlen. Mit der Bestrahlung des gesamten Luftstromes 2 in dem Bereich unmittelbar hinter dem Lüfter 6 ist eine besonders einfache Möglichkeit gegeben, den Luftstrom 2 effektiv und vollständig zu behandeln. Auf diese Weise lassen sich insbesondere Viren, vor allem Corona-Viren, wie Covid-19, oder organische und geruchstragende Substanzen, die nicht über das Abscheideelement 7 abgeschieden wurden, inaktivieren oder reduzieren.

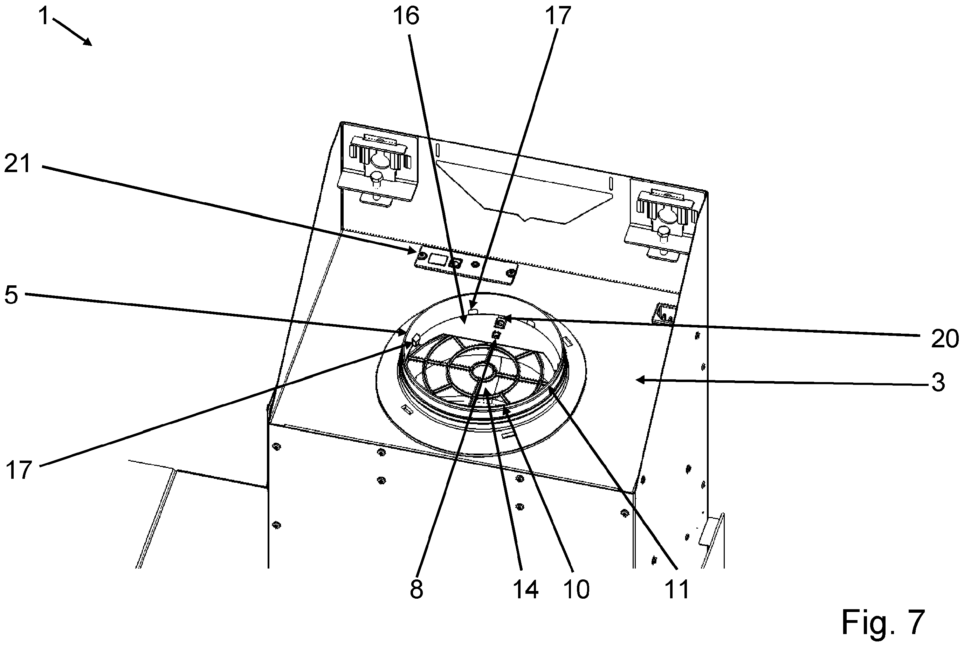

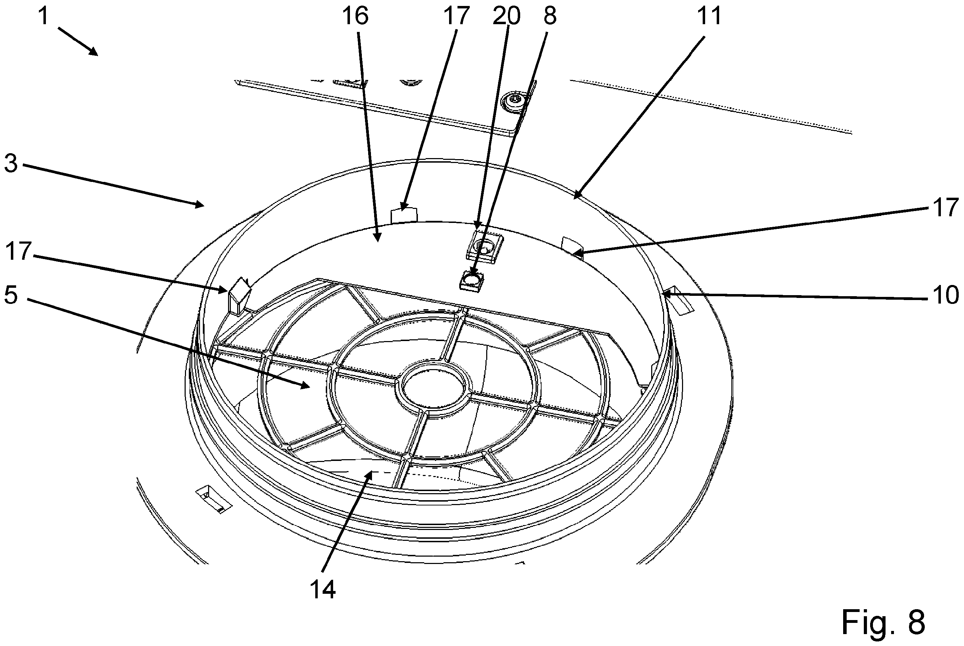

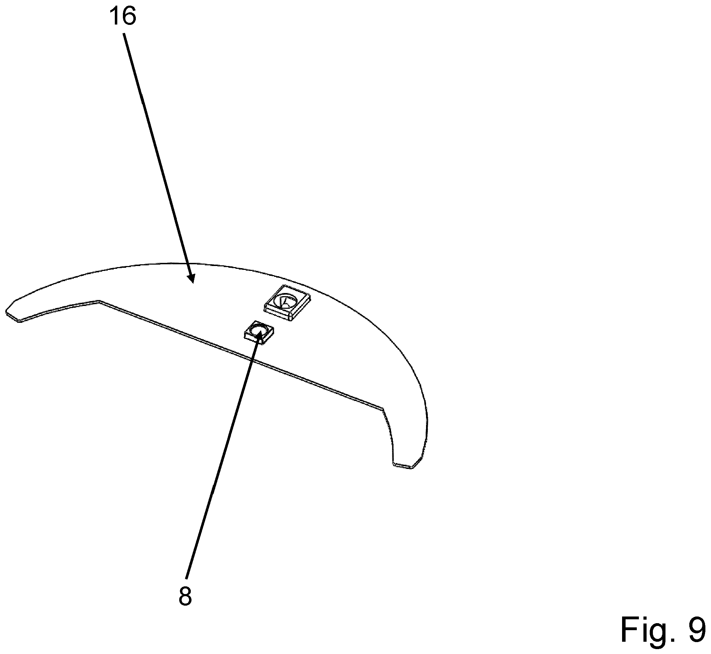

Die Schnittdarstellungen gemäß der Figuren 4, 5, und 6 zeigen außerdem, dass die mindestens eine UVC-Lichtquelle auf einer Platine 16 verlötet in die Ausblasöffnung 14 eingesetzt ist, wobei die UVC-Lichtquelle 8 in Richtung des aus der Ausblasöffnung 14 austretenden Luftstroms 2 abstrahlt. Die Platine 16 ist in Figur 9 in einer Detailansicht einzeln gezeigt. Die Anordnung der UVC-Lichtquelle 8 auf der Platine 16 ermöglicht eine besonders einfache Montage der UVC-Lichtquelle 8, da die Platine 16 einfach in die Ausblasöffnung 14 eingesetzt werden kann. Weiterhin verfügt die Platine 16 über einen Steckverbinder 20 zum Anschluss an eine Spannungsversorgung der Dunstabzugsvorrichtung 1. An dem Gehäuse 3 der Dunstabzugsvorrichtung 1 ist hierzu eine Schnittstellenplatine 21 (Fig. 7) vorgesehen, welche Spannung zum Betrieb der UVC-Lichtquelle 8 zur Verfügung stellt. In den Figuren 4, 5, und 6 sind auch Rasthaken 17 in der Ausblasöffnung 14 zu erkennen, an denen die Platine 16 verrastet ist. Die Verrastung lässt sich besonders gut in Figur 8 erkennen, die eine Detailansicht auf die Ausblasöffnung 14 mit Platine 16 bietet.

Mit der Ausrichtung der Abstrahlrichtung der UVC-Lichtquelle 8 in Richtung der Strömungsrichtung des aus der Ausblasöffnung 14 austretenden Luftstromes 2 kann der Luftstrom 2 zuverlässig beim Ausströmen aus der Ausblasöffnung 14 bestrahlt und Viren, vor allem Corona-Viren, wie Covid-19, oder organische und geruchstragende Substanzen im Luftstrom effektiv und vollständig inaktiviert oder zersetzt werden. Vorteilhafterweise wird der Luftstrom 2 hierzu hinter dem Lüfter 6 in einen mit der UVC-Lichtquelle 8 bestrahlten Bereich geführt, der zur Verlangsamung des Luftstroms 2 einen vergrößerten Querschnitt 9 aufweist.

Damit kann die Verweildauer der Luft des Luftstromes 2 im bestrahlten Bereich erhöht werden, sodass eine effektive Entkeimung mit vergleichsweise geringer Lichtleistung der UVC-Lichtquelle 8 möglich ist. Es können auch mehrere UVC-Lichtquellen 8 dazu eingerichtet sein, den Luftstrom 2 zu bestrahlen. Vorteilhafterweise können mehrere UVC-Lichtquellen 8 dazu eingerichtet sein, einen Abschnitt des Luftstromes 2 zwischen dem Luftauslass 5 und dem Filterelement 15 zu bestrahlen. Die UVC-Lichtquelle 8 ist im Ausführungsbeispiel in einer umfangsseitig geschlossenen, von dem Luftstrom 2 durchströmten Entkeimungszelle 11 angeordnet. Diese Entkeimungszelle 11 kann, wie im Ausführungsbeispiel gezeigt, als umfangsseitig begrenzter Kreisring, Bundkragen oder Zylinder ausgebildet sein.

Die mindestens eine UVC-Lichtquelle 8 ist unmittelbar an der Ausblasöffnung 14 des Lüfters 6 angeordnet, um eine besonders effektive Bestrahlung des Luftstromes 2 zu erreichen. Auf diese Weise kann der gesamte Luftstrom 2, der über die Ansaugöffnung 13 (Fig. 3) des Lüfters 6 angesaugt wird, und über die Ausblassöffnung 14 ausgeblasen wird, vollständig von der UVC-Lichtquelle 8 bestrahlt werden. Damit lassen sich insbesondere Viren, vor allem Corona-Viren, wie Covid-19, oder organische und geruchstragende Substanzen wirksam in dem aus dem Lüfter 6 über die Ausblassöffnung 14 austretenden Luftstrom 2 inaktivieren oder zersetzen.

Die mindestens eine UVC-Lichtquelle 8 ist vorteilhafterweise dazu eingerichtet, das Filterelement 15 von dem Luftauslass 5 her zum Zwecke der Entkeimung des Filterelements 15 und/oder zum Zwecke der Beseitigung organischer und geruchstragender Substanzen auf dem Filterelement 15 zu bestrahlen. Das Filterelement 15 bildet einen Innenraum 18, der von den Filterwänden 19 des Filterelements 15 umschlossen ist. Die Filterwände 19 sind dazu eingerichtet, den durch den Innenraum 18 strömenden Luftstrom 2 zu filtern. Wie in den Figuren 4, 5 und 6 zu sehen ist, bilden die Filterwände 19 eine Doppelwand, die bevorzugt mit Aktivkohle oder einem anderen Material (nicht dargestellt) zur Desodorierung des Luftstromes 2 ausgefüllt sind. Der Innenraum 18 wird also von dem Luftstrom 2 durchströmt und die Filterwände 19 filtern den Luftstrom hiernach. Die mindestens eine UVC-Lichtquelle 8 bestrahlt sowohl die Filterwände 19 des Innenraums 18 als auch den Innenraum 18 selbst. Sowohl die Filterwände 19 des Innenraums 18 als auch der Innenraum 18 selbst werden also von der mindestens einen UVC-Lichtquelle 8 zum Zwecke der Entkeimung von Innenraum 18 und Luftstrom 2 bestrahlt. Die vorherige Bestrahlung des Luftstromes 2 mit der UVC-Lichtquelle 8 ermöglicht es auch, das Adsorptionsvermögen des Geruchsfilters 15 zu verbessern. Der alleinige Einsatz beispielsweise eines mit Aktivkohle versehenen Geruchsfilters 15 ist nur begrenzt geeignet zur Desodorierung des in die Raumluft zurückgeführten Luftstromes 2, da die Fähigkeit der Aktivkohle, geruchstragende Substanzen zu binden, begrenzt ist. Mit der Bestrahlung lassen sich organische und geruchstragende Substanzen aus dem Innenraum 18 und aus dem durch den Innenraum 18 strömenden Luftstrom 2 beseitigen. Über den Innenraum 18 des Filterelements 15 kann der Luftstrom 2 einfach durch einen vergrößerten Querschnitt verlangsamt werden, sodass eine effektive, d.h. über einen hinreichenden Zeitraum andauernde Behandlung des Luftstromes 2 durch die mindestens eine UVC-Lichtquelle 8 in dem bestrahlten Innenraum 18 möglich ist. Das hierbei auf die Filterwände 19 abgestrahlte Licht der UVC-Lichtquelle 8 sorgt zusätzlich für eine Entkeimung der Filterwände 19 und dient zur Beseitigung organischer und geruchstragender Substanzen auf den Filterwänden 19 im Innenraum 18 des Filterelements 15.

### - Bezugszeichenliste -

### Bezuaszeichenliste

- 1: Dunstabzugsvorrichtung
- 2: Luftstromes
- 3: Gehäuse
- 4: Luftansaugöffnung
- 5: Luftauslass
- 6: Lüfter
- 7: Abscheideelement
- 8: UVC-Lichtquelle
- 9: vergrößerter Querschnitt
- 10: Abschnitt
- 11: Entkeimungszelle
- 12: Reflektor
- 13: Ansaugöffnung (Lüfter)
- 14: Ausblasöffnung (Lüfter)
- 15: Filterelement
- 16: Platine
- 17: Rasthaken
- 18: Innenraum
- 19: Filterwände
- 20: Steckverbinder
- 21: Schnittstellenplatine

## Patentansprüche

1. Dunstabzugsvorrichtung (1) zum Abzug von Kochdünsten mittels eines Luftstromes (2), mit
- einem Gehäuse (3), das wenigstens eine Luftansaugöffnung (4) und wenigstens einen Luftauslass (5) aufweist,
- wenigstens einem in dem Gehäuse (3) angeordneten Lüfter (6) zur Erzeugung des Luftstromes (2), und
- wenigstens einem in dem Gehäuse (3) in dem Luftstrom (2) zwischen Luftansaugöffnung (4) und Lüfter (6) angeordneten Abscheideelement (7) zur Trennung eines oder mehrerer Bestandteile, insbesondere Fett und Öl, von den Kochdünsten,
**dadurch gekennzeichnet,**
**dass** in oder an dem Gehäuse (3) mindestens eine UVC-Lichtquelle (8) angeordnet ist, die eingerichtet ist, den aus dem Lüfter (6) austretenden Luftstrom (2) zum Zwecke der Entkeimung des aus dem Lüfter (6) austretenden Luftstroms (2) und/oder zum Zwecke der Beseitigung organischer und geruchstragender Substanzen zu bestrahlen.

2. Dunstabzugsvorrichtung (1) nach Anspruch 1, **dadurch gekennzeichnet, dass** der Luftstrom (2) in dem von der mindestens einen UVC-Lichtquelle (8) bestrahlten Bereich zur Verlangsamung des Luftstroms (2) mit einem vergrößerten Querschnitt (9) geführt ist.

3. Dunstabzugsvorrichtung (1) nach Anspruch 1 oder 2, **dadurch gekennzeichnet, dass** mehrere UVC-Lichtquellen (8) dazu eingerichtet sind, den Luftstrom (2) zu bestrahlen.

4. Dunstabzugsvorrichtung (1) nach Anspruch 3, **dadurch gekennzeichnet, dass** die UVC-Lichtquellen (8) in einer umfangsseitig geschlossenen, von dem Luftstrom (2) durchströmten Entkeimungszelle (11) angeordnet sind.

5. Dunstabzugsvorrichtung (1) nach Anspruch 4, **dadurch gekennzeichnet, dass** die UVC-Lichtquellen (8) innerhalb von einem oder auf einem von dem Luftstrom (2) durchströmten, die Entkeimungszelle (11) umfangsseitig begrenzenden Kreisring oder Zylinder angeordnet sind.

6. Dunstabzugsvorrichtung (1) nach einem der vorhergehenden Ansprüche, **dadurch gekennzeichnet, dass** mindestens ein die von der UVC-Lichtquelle (8) emittierte UVC-Strahlung reflektierender Reflektor (12) in dem Gehäuse (3) angeordnet ist.

7. Dunstabzugsvorrichtung (1) nach Anspruch 6, **dadurch gekennzeichnet, dass** der Reflektor (12) gegenüber mindestens einer UVC-Lichtquelle (8) angeordnet ist, wobei der Luftstrom (2) zur Entkeimung zwischen dem Reflektor (12) und der gegenüberliegenden UVC-Lichtquelle (8) geführt ist.

8. Dunstabzugsvorrichtung (1) einem der vorhergehenden Ansprüche, **dadurch gekennzeichnet, dass** der Lüfter (6) eine Ansaugöffnung (13) und eine Ausblasöffnung (14) aufweist, wobei mindestens eine UVC-Lichtquelle (8) unmittelbar an der Ausblasöffnung (14) angeordnet ist.

9. Dunstabzugsvorrichtung (1) nach Anspruch 8, **dadurch gekennzeichnet, dass** die mindestens eine UVC-Lichtquelle (8) auf einer Platine (16) verlötet in die Ausblasöffnung (14) eingesetzt ist, wobei die UVC-Lichtquelle (8) in Richtung des aus der Ausblasöffnung (14) austretenden Luftstroms (2) abstrahlt.

10. Dunstabzugsvorrichtung (1) nach Anspruch 9, **dadurch gekennzeichnet, dass** die Platine (16) an Rasthaken (17) in der Ausblasöffnung (14) verrastbar ist.

11. Dunstabzugsvorrichtung (1) nach Anspruch 9 oder 10, **dadurch gekennzeichnet, dass** die Platine (16) einen Steckverbinder (20) für eine Spannungsversorgung aufweist.

12. Dunstabzugsvorrichtung (1) nach einem der vorhergehenden Ansprüche, **dadurch gekennzeichnet, dass** dem Luftauslass (5) ein den Luftstrom (2) filterndes Filterelement (15), insbesondere ein Geruchsfilter, nachgeordnet ist.

13. Dunstabzugsvorrichtung (1) nach Anspruch 12, **dadurch gekennzeichnet, dass** die mindestens eine UVC-Lichtquelle (8) dazu eingerichtet ist, das Filterelement (15) von dem Luftauslass (5) her zum Zwecke der Entkeimung des Filterelements (15) und/oder zum Zwecke der Beseitigung organischer und geruchstragender Substanzen auf dem Filterelement (15) zu bestrahlen.

14. Dunstabzugsvorrichtung (1) nach Anspruch 13, **dadurch gekennzeichnet, dass** das Filterelement (15) einen durch Filterwände (19) des Filterelements (15) gebildeten Innenraum (18) aufweist, wobei die Filterwände (19) dazu eingerichtet sind den durch den Innenraum (18) strömenden Luftstrom (2) zu filtern, wobei die Filterwände (19) des Innenraums (18) und der Innenraum (18) von der mindestens einen UVC-Lichtquelle (8) bestrahlt werden.

15. Dunstabzugsvorrichtung (1) nach einem der vorhergehenden Ansprüche, **dadurch gekennzeichnet, dass** die mindestens eine UVC-Lichtquelle (8) eine UVC-Strahlung emittierende Leuchtdiode ist.

16. Dunstabzugsvorrichtung (1) nach einem der vorhergehenden Ansprüche, **dadurch gekennzeichnet, dass** die mindestens eine UVC-Lichtquelle (8) eine UVC-Strahlung mit einer Wellenlänge von 100 bis 280 nm emittiert.

17. Dunstabzugsvorrichtung (1) nach einem der vorhergehenden Ansprüche, **dadurch gekennzeichnet, dass** die mindestens eine UVC-Lichtquelle (8) dazu ausgelegt ist, in dem aus dem Lüfter (6) austretenden Luftstrom (2) Ozon zu erzeugen.
